# EUROPEAN PATENT APPLICATION

(11) **EP 0 774 236 A1**
(43) Date of publication of application: **21.05.1997**
(21) Application number: 95830479.2
(22) Date of filing: 15.11.1995
(51) Int. Cl.: A61B 6/04

(54) **A table for diagnostic examinations**

(71) Applicant: C.A.T. DI CORSINI GIUSEPPE & C. S.p.A., 40044 Sasso Marconi (Bologna) (IT)
(72) Inventor: Sitta, Stefano, I-40044 Pontecchio Marconi (Bologna) (IT)
(74) Representative: Lanzoni, Luciano

(57) **Abstract**

The couch of a table (1) for diagnostic examinations consists in a platform (2) manoeuvrable away from the horizontal to a first limit position (I), obtained by rotation in a first direction (R1) and through a first angle (α) effectively perpendicular to the horizontal, and a second limit position (II) obtained by rotation in a second direction (R2) opposite to the first (R1), through a second angle (β) less than the first angle; other parts of the table include a base frame (3), and, between the platform (2) and the base frame (3), an intermediate frame (4) of which a first end (41) is hinged to a central portion (20) of the platform (2) at a point coinciding with a first pivot axis (X), and a second end (42) is hinged to the base frame (3) at a point coinciding with a second pivot axis (Y); also an actuating and positioning mechanism (5) comprising elements of selectively variable length, interposed between and impinging on the base frame (3) and the platform (2), and a coupling assembly (6) by which the platform (2) and the intermediate frame (4) are either locked together during rotation in the first direction (R1) or disengaged so that the platform (2) can rotate alone in the second direction (R2).

## Description

The present invention relates to a table for use in diagnostic examinations, in particular a table with variable tilt on which to extend a patient.

Various techniques are adopted currently in conducting a range of diagnostic tests, for example radiological examinations on the gastrointestinal tract, the lungs or the urogenital and nervous systems, which involve varying the position of the patient under examination; for this reason, the patient generally is extended on and suitably secured to a special table substantially free to pivot about one or more horizontal axes, in such a manner as will enable rotation about at least one of these axes to maximize the effectiveness of the test.

Reference may be made by way of example to EP 146 006 and EP 244 560, which relate to radiology equipment comprising a table designed for use in situations of the type mentioned above. The underside of the table in question is rigidly associated with a carriage or slide type element presenting the shape (or rather the volume) essentially of a cylindrical sector radiating from a horizontal axis, of which the arcuate surface is directed downwards. This same carriage element is supported from beneath with freedom to pivot about its own axis, and connected thus to a suitable actuating system or mechanism capable of generating movement whereby the patient can be arranged in any position ranging between a substantially upright posture, with head uppermost, and a recumbent position with the body marginally inclined in relation to a horizontal plane and the head at a level slightly lower than that of the feet.

Whilst the patent solutions outlined above may differ one from another in minor details, such as the shape exhibited by certain of the sliding ways, both betray drawbacks deriving from the geometry of the carriage element located beneath the table: the dimensions of this element are such, in effect, by reason of the circumferential portion operating in association with the actuating system or mechanism, as to take up the entire area below the table proper and thus maximize the overall space occupied by the equipment.

Moreover, the association between the carriage element and the table has the effect of increasing the masses brought into play, a factor that dictates the need for an actuating system of considerable power, and at the same time for floor anchorages of massive proportions.

In addition, there is the requirement for particularly complex transmission components such as curvilinear faced gears, which do little to simplify the mechanics of the actuating system.

Accordingly, the object of the present invention is to overcome the drawbacks outlined above.

The stated object is realized according to the present invention in a table for diagnostic examinations that comprises a platform, associated pivotably with a base and rotatable thus about two fulcrum axes, also linear actuating and positioning means of which the length is variable and the platform thereby rendered capable of movement between two stable limit positions: a first, inclined in relation to a horizontal reference plane and obtained by rotation in a first direction, and a second obtained by rotation in a direction opposite to the first direction.

Advantageously, a more compact transverse dimension is achieved according to the present invention, thanks to the adoption of actuating means that do not project from the bearing structure of the table; additional advantage is gained, moreover, by the use of actuating means which operate in a single plane and are capable of holding a selected position without generating any oscillation whatever in the table once at the desired angle of inclination.

The invention will now be described in detail, by way of example, with the aid of the accompanying drawings, in which:
- figs 1, 2 and 3 show three positioning possibilities afforded by a table embodied in accordance with the present invention, illustrated in a side elevation with certain parts enlarged;
- fig 4 illustrates the table of figs 1-3, viewed in plan from above and on a different scale, with certain parts cut away better to reveal others;
- fig 5 illustrates a part of the table as in figs 1-4, viewed in section.

With reference to the drawings, the invention relates to a table for diagnostic examinations, denoted 1 in its entirety, which is shown in a preferred embodiment albeit implying no limitation.

The table 1 affords a platform 2 with a predominating longitudinal dimension, on which to extend a patient for examination.

In view of the different types of examinations to be performed and the different methods employed in their implementation, the table 1, or rather the platform 2, can be disposed at different angles of inclination, and in particular, moved between two limit positions.

The first such limit position is obtained by rotating the platform 2 from the horizontal through a first direction of rotation, denoted R1, whilst the second limit position is obtained likewise by rotating the platform 2 away from the horizontal, in this instance through a second direction R2 opposite to the first.

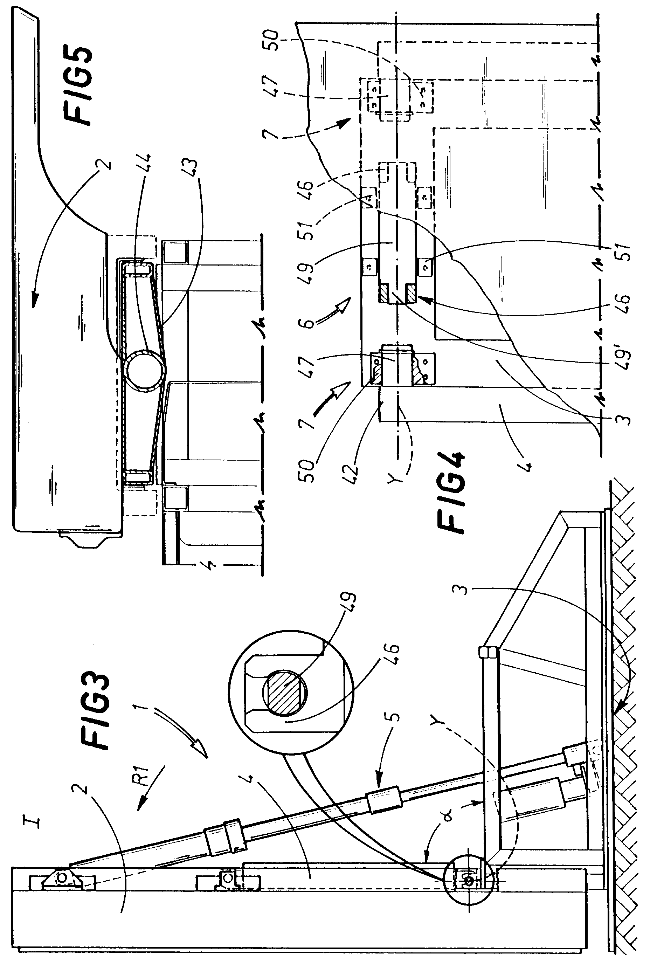

In the particular example illustrated, the first limit position is denoted I and indicated in fig 3, where the platform 2 will be seen to occupy a substantially vertical position, i.e. disposed at an angle α of 90° relative to the horizontal.

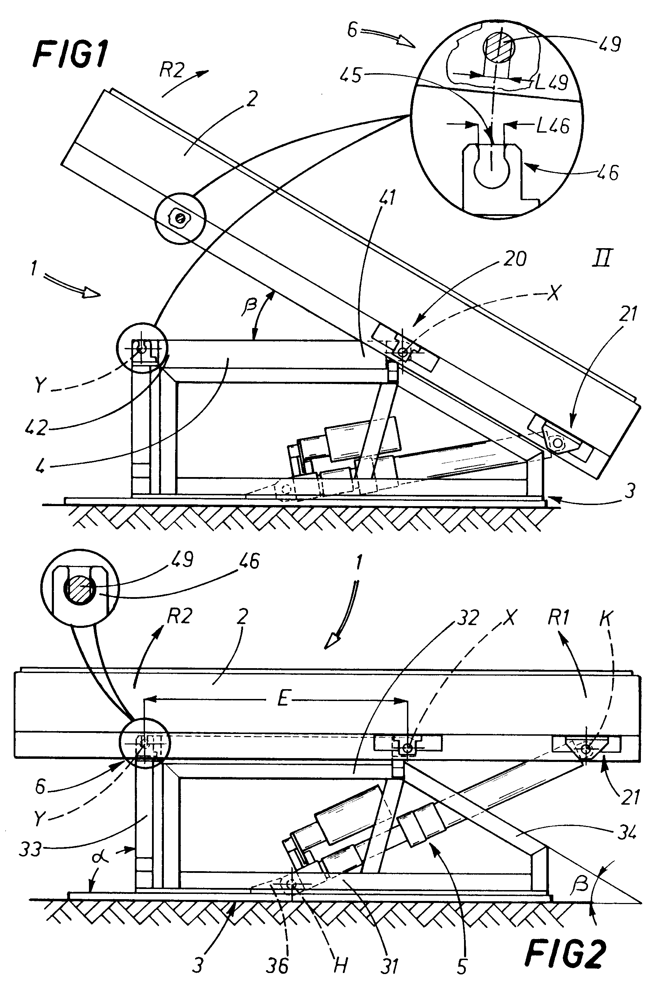

In the second limit position, denoted II and indicated in fig 1, the platform 2 is inclined at -30°, that is to say disposed at an angle β of 30° with respect to the horizontal, obtained by rotation in the direction opposite to the direction producing the first limit position I. Observing figs 1, 2 and 3, more exactly, the first direction of rotation R1 is anticlockwise and the opposite second direction of rotation R2 clockwise.

Needless to say, these same directions of rotation and angles of inclination are referred to the particular example illustrated.

The manner of positioning the table 1, or rather the structural components necessary to produce the various movements, will be described in due course.

The table 1 substantially comprises the platform 2, as already intimated, also a supporting or base frame 3, an intermediate frame 4, actuating and positioning means 5, and releasable connection means 6, all of which to be described in due course.

The base frame 3 is anchored rigidly to the floor and will be seen in the example illustrated to exhibit a substantially right trapezoidal profile comprising a longer horizontal side 31 positioned to lie flat on the floor, and a shorter horizontal side 32 extending for a distance substantially equal to the length of the intermediate frame 4 (described in due course).

Also forming part of the right trapezoidal profile are a vertical side 33 and an oblique side 34, disposed respectively at 90° and at 30° in relation to the two horizontal sides.

The intermediate frame 4 is located between the base frame 3 and the platform 2.

As discernible from the cross section of fig 5, the intermediate frame 4 essentially comprises an outer box member 43, and an internal tubular member 44 of circular section disposed centrally in the box member; this particular structure is especially effective in withstanding both torsional and bending stresses.

A first end 41 of the intermediate frame 4 (indicated in fig 1) is pivotably associated with a substantially central area 20 of the platform 2, and a second end 42 pivotably associated with the base frame 3, at points coinciding respectively with a first fulcrum axis X and a second fulcrum axis Y disposed horizontally and extending transversely to the longitudinal dimension of the platform 2.

Referring to fig 4, in which 7 denotes the pivotable connection between the intermediate frame 4 and base frame 3, the second end 42 of the intermediate frame 4 affords two distinct extremities presenting respective pivots 47. The pivots 47 are anchored stably to the base frame 3 by means of suitable fixing sleeves 50, in such a way that the intermediate frame 4 remains permanently associated with the base frame 3 and at the same time rotatable about the second axis Y.

Thus, in practice, the intermediate frame 4 is hinged by its two ends 41 and 42 to the remaining parts of the table, namely the platform 2 and the base frame 3, at points coinciding respectively with the two fulcrum axes X and Y.

The aforementioned actuating and positioning means 5 comprise elements of which the length can be varied at will. Such means 5 might consist, for instance, in a plurality of elements connected one with another by lead screws and lead nuts or indeed other means of any given type provided that these afford the possibility of extending and retracting from one stable position to another.

The actuating and positioning means 5 are interposed between and designed to interact with the base frame 3 and a portion 21 of the platform 2; in the example of the drawings, this same portion 21 is an end portion of the platform 2 located outside the area compassed by the distance E between centres of the two fulcrum axes X and Y.

More particularly, such means 5 might be associated pivotably with a bottom portion 36 of the base frame 3 in such a way as to allow rotation about a horizontal axis, denoted H, and similarly with the aforementioned end portion 21 of the platform 2, allowing rotation about a further horizontal axis K, so that the effect of extending the actuating and positioning means 5 is to distance the end portion 21 of the platform 2 from the bottom portion 36 of the base frame 3. Conversely, these same two portions are drawn together by reducing the length of the actuating and positioning means 5.

The structure of the releasable connection means 6, as applicable to the embodiment of the table illustrated by way of example, will be described in due course.

From an operational standpoint, the function of the releasable connection means 6 is to ensure that the intermediate frame 4 and the platform 2 remain coupled in mutually parallel association during rotation in the first direction R1, whilst allowing the platform 2 to uncouple from the intermediate frame 4 when rotated in the second direction R2.

Thus, when the table 1 is manoeuvred into the first limit position I (assuming an angle of +90° in the example illustrated), the intermediate frame 4 and the platform 2 are caused to rotate together as one about the second fulcrum axis Y. In effect, the intermediate frame 4 will separate from the shorter side 32 of the base frame 3 and remain associated with the platform 2 both during rotation about the horizontal axis Y and when occupying the stable position ultimately assumed (vertically disposed in fig 3).

When the table 1 is substantially level as in fig 2, with both the intermediate frame 4 and the platform 2 horizontally disposed, the connection means 6 occupy an intermediate configuration, that is, a position from which the platform 2 and the intermediate frame 4 can be either locked together and rotated thus as one through the first direction R1, or separated to allow the rotation of the platform 2 alone in the opposite direction R2.

For the table 1 to assume the second limit position II (with the platform angled at -30° from the horizontal in the case of the example illustrated), in fact, the releasable connection means 6 must uncouple in such a manner that the intermediate frame 4 can separate from the platform 2, while remaining pivotably associated with the base frame 3 at the end denoted 42, leaving the platform 2 free to rotate about the first axis X.

Accordingly, the table according to the invention is one utilizing two distinct axes on which to centre two different directions of rotation.

The releasable connection means 6 might be structured differently yet to equally good effect, provided they fulfil the requirement of anchoring the intermediate frame 4 and platform 2 together during rotation in the first direction R1, and releasing the platform 2 when rotated in the second direction R2 toward the second limit position II.

Whilst in the example of the drawings the connection means 6 occupy a position coinciding with the second axis Y, such means 6 might obviously be positioned differently in other solutions, always providing that the intermediate frame 4 and the platform 2 can be locked together in the manner described above.

From the structural standpoint, again with reference to the example illustrated, the connection means 6 consist in a coupling element 49 associated with an underside portion of the platform 2 and a matching socket 46 afforded by the base frame 3.

The socket 46 might equally well be afforded by the intermediate frame 4 in an alternative embodiment of the invention, not illustrated in the drawings, given that to the end of obtaining a pivotable association between intermediate and base frames 4 and 3 centred on the second fulcrum axis Y, the effect of coupling the platform 2 and the intermediate frame 4 along this axis Y is the same as that of coupling the platform 2 and the base frame 3.

With reference to fig 4, in particular, the coupling element could be embodied as a horizontally disposed pivot 49 of which a central portion is secured to the platform 2 by means of fixing elements 51, consisting for example in shackles positioned over the pivot 49 and offered to the bottom face of the platform 2.

Each end 49' of the pivot 49 presents a substantially circular cross section reduced by a pair of parallel chords, as discernible from the enlarged insets of figs 1, 2 and 3. The two chords serve to establish two flat lands, separated by a first distance L49, which are vertically disposed when the platform 2 occupies the horizontal position as in fig 2.

The socket 46 affords a matching hollow section that appears essentially circular and terminates uppermost in an opening 45 of width L46 marginally greater than the width L49 of the coupling element.

Thus, the end 49' of the pivot 49 can locate in and disengage from the socket 46 only when the platform 2 occupies a substantially horizontal position, with the pivot 49 and the socket 46 arranged as indicated in the enlarged inset of fig 2.

As the actuating and positioning means 5 are extended, causing the platform 2 in consequence to rotate about the second axis Y toward the first limit position I (+90° in the example illustrated), the ends 49' of the pivot 49 will be restrained by the sockets 46 in such a way that the intermediate frame 4 and the platform 2 remain locked one to another while able to rotate as one about the second axis Y.

Conversely, when the actuating and positioning means 5 are retracted, causing the platform 2 to rotate in the second direction R2, the two ends 49' of the pivot 49 will disengage from the sockets 46, separating by way of the respective openings 45. In this situation, the platform 2 is uncoupled from the second fulcrum axis Y and thus able to pivot about the first fulcrum axis X, hence toward the second limit position II (-30° in the example illustrated).

## Claims

1. A table for diagnostic examinations, of the type with a platform (2) exhibiting a predominating longitudinal dimension and affording a couch on which to extend a patient, wherein the platform (2) is positionable at different angles of inclination and in particular capable of movement between a first limit position (I) obtained by rotation in a first direction (R1) and through a first angle (α), approximating to a right angle in relation to the horizontal, and a second limit position (II) obtained by rotation in a second direction (R2), opposite to the first direction (R1), through a second angle (β) less than the first angle, characterized
in that it comprises:
- a base frame (3);
- an intermediate frame (4) interposed between the platform (2) and the base frame (3), of which a first end (41) is pivotably associated with a substantially central area (20) of the platform (2) at a point coinciding with a first horizontally disposed fulcrum axis (X) extending transversely to the longitudinal dimension of the platform (2), and a second end (42) is pivotably associated with the base frame (3) at a point coinciding with a second horizontally disposed fulcrum axis (Y) disposed parallel to the first;
- releasable connection means (6), by which the platform (2) is caused to remain stably in parallel association with the intermediate frame (4) when rotated in the first direction (R1), and allowed movement independently of the intermediate frame (4) when rotated in the second direction (R2);
- actuating and positioning means (5) consisting in elements of selectively variable length interposed between and interacting with the base frame (3) and the platform (2), by which the platform (2) is caused to rotate in the first direction (R1) about the second fulcrum axis (Y) or in the second direction (R2) about the first fulcrum axis (X).

2. A table for diagnostic examinations as in claim 1, wherein the releasable connection means (6) are located to coincide with the second fulcrum axis (Y).

3. A table for diagnostic examinations as in claim 1, wherein the releasable connection means (6) serve to couple the platform (2) and the intermediate frame (4) stably together when rotated about the second fulcrum axis (Y), and the actuating and positioning means (5) are interposed between and designed to interact with the base frame (3) on the one hand, and on the other, an end portion (21) of the platform (2) lying outside an area compassed by the distance (E) between centres of the first fulcrum axis (X) and the second fulcrum axis (Y).

4. A table for diagnostic examinations as in claim 1, wherein the releasable connection means (6) comprise a coupling element (49) associated with an underside portion of the platform (2) and a matching socket (46) afforded by the base frame (3).

5. A table for diagnostic examinations as in claim 1, wherein the releasable connection means (6) comprise a coupling element (49) associated with an underside portion of the platform (2) and a matching socket (46) afforded by the intermediate frame (4).

6. A table for diagnostic examinations as in claim 4 or claim 5, wherein the coupling element consists in a horizontal pivot (49), disposed coaxially with the second fulcrum axis (Y) at least when the platform (2) lies horizontally disposed, of which a central portion is secured to the platform (2) and of which the two ends (49') each present a substantially circular cross section reduced in width by a pair of parallel chords serving to establish two flat lands separated by a first distance (L49), which are vertically disposed when the platform (2) is horizontally disposed, whilst the matching socket (46) affords a hollow section of essentially circular profile terminating uppermost in an opening (45) of width (L46) marginally greater than the width established by the first distance (L49), in such a way that the end (49') of the pivot (49) can locate in and disengage from the socket (46) only when the platform (2) occupies a substantially horizontal position.

7. A table for diagnostic examinations as in claim 1, wherein the base frame (3) exhibits a substantially trapezoidal profile of which one horizontal side (32) is disposed uppermost and the two adjoining sides (33, 34) are disposed at angles corresponding respectively to the first angle (α) and to the second angle (β), in such a way as to establish locating surfaces offered to the platform (2) when rotated respectively into the first limit position (I) and into the second limit position (II).

8. A table for diagnostic examinations as in claim 1, wherein the intermediate frame (4) exhibits a cross sectional profile appearing as an outer box member (43) and an internal tubular member (44) of circular section disposed centrally within the box member.
